(19) Europäisches Patentamt
European Patent Office
Office européen des brevets

(11) Publication number: **0 009 996**
**B1**

(12) **EUROPEAN PATENT SPECIFICATION**

(45) Date of publication of patent specification: **23.02.83**

(51) Int. Cl.[3]: **C 07 D 519/04**

(21) Application number: **79302152.8**

(22) Date of filing: **09.10.79**

(54) **Preparation of vindesine monosulfate.**

(30) Priority: **10.10.78 US 949595**

(43) Date of publication of application:
**16.04.80 Bulletin 80/8**

(45) Publication of the grant of the patent:
**23.02.83 Bulletin 83/8**

(84) Designated Contracting States:
**DE GB IT NL SE**

(56) References cited:
DE - A - 2 558 027
DE - A - 2 630 392
DE - A - 2 739 443

(73) Proprietor: **ELI LILLY AND COMPANY**
**307, East McCarty Street**
**Indianapolis Indiana 46285 (US)**

(72) Inventor: **Rolski, Stanislaw**
**4329 "B" Lakeway Drive**
**Indianapolis, Indiana 46205 (US)**

(74) Representative: **Hudson, Christopher Mark et al,**
**European Patent Attorney Erl Wood Manor**
**Windlesham Surrey GU20 6PH (GB)**

**EP 0 009 996 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Preparation of vindesine monosulfate

Vindesine is disclosed in the Belgian Patent No. 813,168 of Cullinan, and Gerzon, issued October 2, 1974, and is named therein as 4-desacetyl VLB C—3 carboxamide. The compound has the following structure

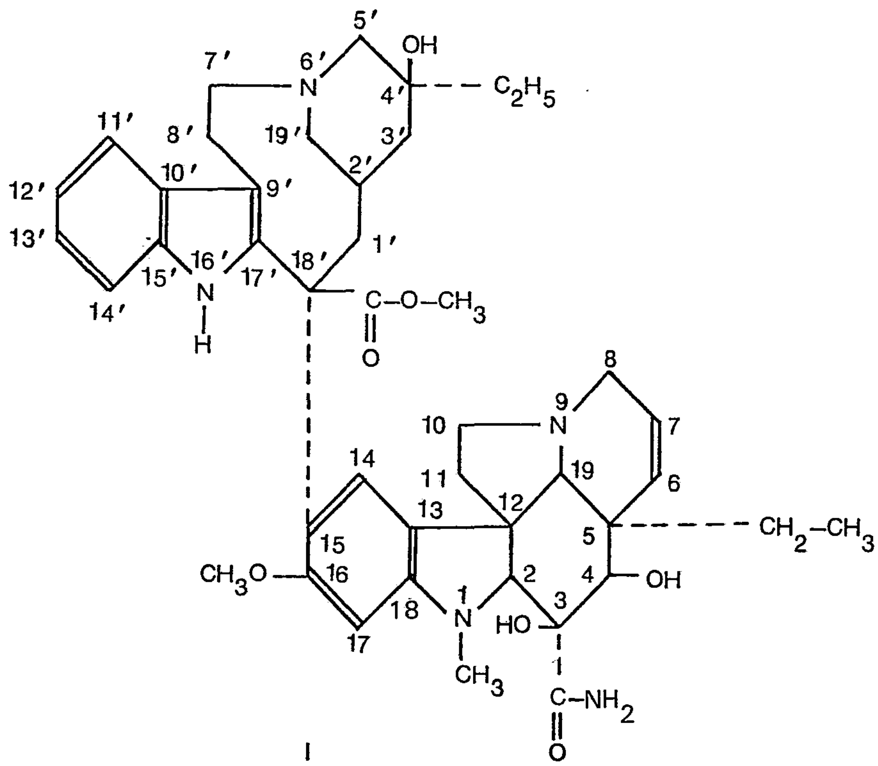

I

Vindesine can also be named as 4-desacetyl-3-descarbomethoxy- VLB 3-carboxamide or as 23-amino-$O^4$-desacetyl-23-demethoxy-vincaleukoblastine. A sulfate salt of vindesine is also specifically disclosed in Belgian Patent No. 813,168 issued October 2, 1974.

In transplanted tumor systems in animals, vindesine has shown an activity approaching that of vincristine although potentially lacking some of the neurological side-effects which accompany the clinical use of the latter drug. Vindesine is now undergoing a world-wide clinical trial and has found utility in early tests against certain leukemias, lymphomas, and some carcinomas of the lung.

Vinblastine and vincristine two related *vinca* alkaloids now being marketed as oncolytic agents, are formulated as sulfate salts for use in intravenous administration to patients suffering from various tumors. It was, however, found that vindesine sulfate prepared by simply mixing a solution of vindesine base in different solvents with 2 equivalents of dilute sulfuric acid and then evaporating to dryness was electrostatic (difficult to handle) and its stability was unsatisfactory for storage.

This invention provides a method for the preparation of vindesine mono sulfate which yields a product of good shelf stability which is easy to handle.

This invention provides a process for the preparation of vindesine sulfate which comprises the steps of dissolving substantially pure vindesine base in acetonitrile or acetone at a concentration of about 1% and then adding thereto one mole of 1N aqueous sulfuric acid with stirring at a rate of 1 ml per minute or slower such as to minimize formation of vindesine disulfate to yield a product which is substantially pure vindesine monosulfate.

The rate of addition and degree of mixing becomes critical as the neutralisation of the vindesine base nears completion.

In my novel process, the preparation of the mono-sulfate salt of vindesine is preferably carried out in two steps. First, one equivalent of 1N aqueous sulfuric acid is added slowly (dropwise with stirring) to a 1% vindesine solution in acetonitrile. After this addition has been completed, the resulting suspension is stirred for about 1/2 hour after which time, the slow addition with stirring of the remaining equivalent of 1N aqueous sulfuric acid is carried out. Stirring is then continued for an additional half-hour, after which time the precipated vindesine mono sulfate is separated by filtration. The filter cake is washed with the same organic solvent used to dissolve the vindesine base initially, and is then dried to a constant weight. The yield of mono sulfate from base is 94—96 percent.

More specifically, the 1N aqueous sulfuric acid is added to the 1% solution of vindesine in acetonitrile, at a rate such that the rate of addition of sulfuric acid to the vindesine solution coupled with the rate of stirring or degree of mixing serves to avoid the formation of vindesine disulfate. A rate of about 1 ml./min. has been found to be satisfactory, particularly towards the end of the neutralization process. A buret is conveniently employed for the addition.

In carrying out the above process the chief product formed between vindesine base and the first equivalent of sulfuric acid is the hemisulfate (2 moles of vindesine per mole of sulfuric acid). The second equivalent of sulfuric acid, which is added at the same rate, converts the hemisulfate to the monosulfate (one mole of vindesine per mole of sulfuric acid). If the addition is too rapid, especially toward the end of the neutralization process or if excess sulfuric acid is used, disulfate (one mole of vindesine per 2 moles of sulfuric acid) is formed. If the amount of sulfuric acid added to the vindesine is calculated as 2 equivalents based upon the weight of vindesine taken as 97% pure (pure alkaloid containing solvent, etc.) any sulfuric acid that is tied up as disulfate leaves, by necessity, some vindesine present as hemisulfate since there is insufficient sulfuric acid to form a monosulfate for the entire amount of vindesine. The hemisulfate is less stable than vindesine monosulfate. For example, at 25°C. for one month, vindesine hemisulfate has only 85.2% of initial potency, but vindesine monosulfate 97.4% of initial potency. Thus, the slow addition process of this invention avoids disulfate formation and the presence of hemisulfate in the product and yields a vindesine monosulfate of satisfactory stability and which is easy to handle for pharmaceutical formulations. The vindesine monosulfate thus produced also has suitable physical properties for use as an analytical standard.

A titration curve for the neutralization of vindesine base with 1N aqueous sulfuric acid carried out as above shows an inflection point at pH = 6.5 corresponding to the formation of vindesine hemisulfate. Addition of the second equivalent of 1N sulfuric acid causes the apparent pH of the solution to drop to a pH in the range 4.5—5.0.

While it is possible to follow the above reaction with a pH meter, the endpoint has proved to be variable (4.5—5.0). It is preferred that the amount of sulfuric acid added is calculated on the basis of a high pressure liquid chromatographic determination of vindesine base present rather than relying upon an endpoint determination. It is very important for the stability of vindesine monosulfate prepared by the process of this invention that vindesine base of high purity be used.

Addition of excess sulfuric acid causes the apparent pH to drop further as the monosulfate is converted to disulfate. The presence of disulfate in monosulfate yields, upon solution (10 mg/ml.) in water, a more acidic pH than a pure vindesine monosulfate solution would yield (pH = 4.5). Vindesine disulfate upon solution in water (10 mg/ml.) has a pH = 2.0. Microanalysis of the solid sulfate shows a higher percent of sulfur than 3.76%, the calculated amount for vindesine monosulfate.

Vindesine monosulfate obtained by the above procedure is hydrated and contains from 4 to 5% water (about 2 moles) by a Karl Fisher determination. Thus, vindesine sulfate contains per molecules 1 mole of vindesine, 1 mole of sulfuric acid and 2 moles of water.

In carrying out the process of this invention, acetonitrile has been specified as the solvent of choice for dissolving vindesine for its neutralization by aqueous sulfuric acid. Acetone can also be used, but acetonitrile is preferred because vindesine monosulfate prepared in that solvent has better stability. Use of 1N sulfuric acid and 1% vindesine solution in acetonitrile (or acetone) has been specified since these concentrations have been found to be optional. As will be apparent to those skilled in the art, manipulation of the above concentrations can produce vindesine monosulfate of satisfactory properties. Such operative processes would fall within the scope of this invention.

This invention is further illustrated by the following specific example.

Example 1

A solution is prepared from 32.84 g. of vindesine base and 3284 ml. of acetonitrile. One equivalent of 1N aqueous sulfuric acid is added thereto with stirring at the rate of 1 ml./min. (1 g. of vindesine free base of 97 percent purity requires 1.25 ml. of 1.0 N aqueous sulfuric acid). After the first equivalent of sulfuric acid has been added, the resulting suspension is stirred at room temperature for a period of from 20 to 30 minutes, after which time the second equivalent of 1N aqueous sulfuric acid is added at the same rate with stirring. During the addition of the second equivalent of sulfuric acid, the hemisulfate formed by the addition of the first equivalent of sulfuric acid is converted to vindesine monosulfate. After the second equivalent of sulfuric acid has been added, the resulting suspension is stirred for an additional 30 minutes at ambient temperature. Vindesine sulfate thus prepared is next filtered and the filter cake was washed with 100 ml. acetonitrile. The precipitate is dried in vacuo at a temperature not exceeding 35°C. to constant weight; yield = 3.4—35.6 g. (94—96%).

In the above example, the rate of addition of dilute sulfuric acid to the vindesine base or vindesine hemisulfate solution was 1 ml./min. Slower rates of addition give substantially the same results. More rapid rates of addition can be utilized provided that stirring is sufficient to insure that the formation of disulfate is avoided. What is important is not the absolute rate of addition of the dilute aqueous sulfuric acid, but addition at a suitable rate coupled with adequate stirring such as to minimize disulfate formation, especially toward the end of the neutralization process.

The above procedure can be carried out in two steps; i.e., the hemisulfate, which is a well-

characterized compound, can be isolated, dried and then resuspended in an organic solvent for the second equivalent of sulfuric acid to be added to the suspension.

**Claims**

1. A process for the preparation of vindesine monosulfate characterized by the steps of dissolving substantially pure vindesine base in acetonitrile or acetone at a concentration of about 1% and then adding thereto one mole of 1N aqueous sulfuric acid with stirring at a rate of 1 ml per minute or slower such as to minimize formation of vindesine disulfate to yield a product which is substantially pure vindesine monosulfate.

2. A process according to Claim 1 in which the solvent is acetonitrile.

3. The process according to Claim 1 characterized by the steps of dissolving vindesine base in acetonitrile or acetone at a concentration of about 1% and then adding thereto one equivalent of 1N aqueous sulfuric acid with stirring so as to form vindesine hemisulfate and then adding a second equivalent of 1N aqueous sulfuric acid thereto with stirring to convert the vindesine hemisulfate to vindesine monosulfate at such a rate as to minimize the formation of vindesine disulfate, to yield a product which is substantially pure vindesine monosulfate as the dihydrate.

**Revendications**

1. Procédé de préparation de monosulfate de vindésine caractérisé par les étapes opératoires consistant à dissoudre la vindésine base substantiellement pure dans l'acétonitrile ou dans l'acétone à raison d'une concentration d'environ 1% et à y ajouter ensuite, en agitant, une mole d'acide sulfurique aqueux 1 N à une vitesse d'l ml par minute ou plus lentement de manière à réduire au minimum la formation de disulfate de vindésine afin d'obtenir un produit qui consiste en monosulfate de vindésine substantiellement pure.

2. Procédé suivant la revendication 1 caractérisé en ce que le solvent est de l'acétonitrile.

3. Procédé suivant la revendication 1 caractérisé par les étapes opératoires consistant à dissoudre une vindésine base dans l'acétonitrile ou dans l'acétone à raison d'une concentration d'environ 1%, à y ajouter ensuite, en agitant, un équivalent d'acide sulfurique aqueux 1 N de manière à former de l'hémisulfate de vindésine et à y ajouter ensuite, en agitant, un second équivalent d'acide sulfurique aqueux 1 N pour transformer l'hémisulfate de vindésine en monosulfate de vindésine à une vitesse telle qu'elle réduise au minimum la formation de disulfate de vindésine afin d'obtenir un produit qui consiste en monosulfate de vindésine substantiellement pure sous forme de dihydrate.

**Patentansprüche**

1. Verfahren zur Herstellung von Vindesinmonosulfat, dadurch gekennzeichnet, daß man praktisch reine Vindesinbase in Acetonitril oder Aceton mit einer Konzentration von etwa 1% löst und die Lösung dann unter Rühren bei einer Zugabegeschwindigkeit von 1 ml/min oder darunter mit 1 Mol 1n wässriger Schwefelsäure versetzt, wodurch man unter nur möglichst geringer Bildung von Vindesindisulfat zu praktisch reinem Vindesinmonosulfat als Produkt gelangt.

2. Verfahren nach Anspruch 1, dadurch gekennzeichnet, daß man als Lösungsmittel Acetonitril verwendet.

3. Verfahren nach Anspruch 1, dadurch gekennzeichnet, daß man Vindesinbase in Acetonitril oder Aceton mit einer Konzentration von etwa 1% löst und die Lösung dann zur Bildung von Vindesinhemisulfat zunächst unter Rühren mit einem Äquivalent 1n wässriger Schwefelsäure versetzt und anschließend zue Umwandlung des Vindesinhemisulfats in Vindesinmonosulfat unter Rühren ein zweites Äquivalent 1n wässriger Schwefelsäure zugibt, wobei man die Zugabegeschwindigkeit so einstellt, daß es zu einer möglichst geringen Bildung von Vindesindisulfat kommt und als Produkt praktisch reines Vindesinmonosulfat in Form des Dihydrats gebildet wird.